Europäisches Patentamt

(19) European Patent Office

Office européen des brevets

(11) Publication number: **0 106 231**
A1

# (12) EUROPEAN PATENT APPLICATION

(21) Application number: 83109725.8

(22) Date of filing: 29.09.83

(51) Int. Cl.³: **C 07 H 15/22**

(30) Priority: 08.10.82 US 433485

(43) Date of publication of application:
25.04.84 Bulletin 84/17

(84) Designated Contracting States:
BE CH DE FR GB IT LI NL SE

(71) Applicant: ABBOTT LABORATORIES
14th Street and Sheridan Road
North Chicago, Illinois 60064(US)

(72) Inventor: Gracey, Howard Eugene
234 Prospect Avenue
Mundelein Illinois 60060(US)

(74) Representative: Modiano, Guido et al,
MODIANO, JOSIF, PISANTY & STAUB Modiano &
Associati Via Meravigli, 16
I-20123 Milan(IT)

(54) Process for the O-demethylation of aminoglycoside compounds.

(57) An improved process for the O-demethylation of O-methyl-containing aminoglycoside compounds by reacting an O-methyl-containing aminoglycoside compound with boron tris(haloacetate).

EP 0 106 231 A1

Croydon Printing Company Ltd.

This invention relates to aminoglycoside antibiotic compounds, and more particularly to an improved process for the O-demethylation of O-methyl-containing aminoglycosides.

The aminoglycoside antibiotics are a valuable therapeutic class of antibiotics which are useful in the treatment of susceptible bacterial infections.

Once an aminoglycoside antibiotic has been in clinical use for a period of time, microorganisms resistant to the antibiotic's growth inhibition activity may develop. In many cases, the resistance is R-factor mediated and is attributed to the ability of the microorganism to enzymatically modify the amino or hydroxyl groups of the antibiotic, and thereby reduce or eliminate its growth inhibition properties. Thus, there is always a need for new chemical entities which can be held in reserve to combat bacterial strains which have become resistant to treatment by clinically used aminoglycoside antibiotics. In the past, it has been found that the antibacterial and pharmacological properties of many naturally produced aminoglycoside antibiotics can be altered by structural modifications. As an example, certain chemical modifications in the gentamicin and kanamycin families of aminoglycoside antibiotics provide structures which are less toxic than the parent antibiotic. Further, in the same series, certain structural modifications alter the antibacterial spectrum advantageously either by increasing the antibiotic's intrinsic activity or by increasing the antibiotic's activity against resistant strains.

As with the gentamicin and kanamycin families of antibiotics, it previously has been determined that chemical modification of fortimicin (also called astromicin) aminoglycoside compounds can result in derivative compounds which exhibit reduced toxicity or increased antibacterial activity with respect to particular susceptible microorganisms; or which exhibit equivalent or reduced activity, but nevertheless are useful as reserve antibiotics in the event resistant strains should develop after a period of clinical use of one or more of the fortimicin antibiotics. One such chemical modification of the parent fortimicin compounds that has received attention is 3-O-demethylation. For example, United States Patent No. 4,124,756 discloses reacting fortimicin B, or other appropriate derivatives containing the fortamine moiety, with excess metallic lithium in an amine solvent, such as ethylamine or ethylenediamine, to obtain, e.g., 3-O-demethylfortimicin B. The 3-O-demethylated compound may then be 4-N-acylated or -alkylated to obtain other useful 3-O-demethylfortimicin B derivatives. As a further example, United States Patent No. 4,187,297 discloses other 2'-N-acyl- and 2'-N-alkyl-3-O-demethylfortimicin B derivatives which are produced by a similar method.

While the foregoing 3-O-demethylfortimicin compounds have been found to exhibit highly desirable properties, the methods disclosed in the foregoing patents for obtaining these compounds result in relatively low yields, and in slow and costly production. In order to overcome these problems, various alternative processes for producing 3-O-demethylfortimicins previously have been proposed. For example, United States Patent No. 4,220,756 discloses reacting lithium wire with ethylenediamine under an inert atmosphere at a temperature of about 8° to about 116°C. until a deep blue color appears, and thereafter stirring fortimicin B in the solution at room temperature until the blue color disappears to obtain 3-O-demethylfortimicin B in a yield of about 54%.

United States Patent No. 4,242,503 discloses reacting a fortimicin with hydroiodic acid or trimethylsilyl iodide to obtain the corresponding 3-O-demethylfortimicin in a yield of about 10 to 30%. United States Patent No. 4,230,848 discloses reacting a fortimicin with a boron trihalide, preferably boron tribromide, in an inert solvent, at a temperature of about 4°C., removing residual solvent and boron trihalide, and then isolating the desired product by chromatography to obtain the corresponding 3-O-demethylfortimicin in a yield of about 30 to about 40%. United States Patent No. 4,330,673 additionally discloses employing this technique in the O-demethylation of other O-methyl-containing aminoglycoside compounds.

While the O-demethylation processes disclosed in these latter patents represent an improvement in yield over the O-demethylation process of United States Patent No. 4,124,756, they still result in relatively low yields on the order of about 10 to 50%, and remain costly and inefficient processes for producing the desired compounds.

It has now been determined that O-methyl-containing aminoglycoside compounds can be O-demethylated in improved yields, and frequently in yields exceeding 60%, by reacting an O-methyl-containing aminoglycoside compound with a boron tris(haloacetate).

The process of the invention is useful in the production of O-demethylated aminoglycoside compounds having antibiotic activity; in the production of O-demethylated aminoglycoside compounds used as intermediates in the production of semi-synthetic aminoglycoside antibiotics; and for other purposes.

In accordance with the process of the invention, an O-methyl-containing aminoglycoside compound is reacted with a boron tris(haloacetate) to obtain the corresponding O-demethylated compound.

For purposes of illustration, the inventive concepts will be hereinafter described in detail in connection with the O-demethylation of fortimicin compounds. However, it is contemplated that the invention may be employed in connection with the O-demethylation of any aminoglycoside compounds containing an O-methyl group which remain stable upon mild acid treatment. Illustrative examples of such other aminoglycoside compounds which may be employed in the practice of the invention include, without limitation, the istamycins, the sannamycins, the dactimicins, the sporaricins, the seldomycins, such as seldomycin factor 5 and like O-methyl-containing compounds. In the case of dactimicin, the O-demethylated derivative produced in accordance with the invention is 3-O-demethylfortimicin A. The fortimicin compounds which can be O-demethylated in accordance with the invention include, without limitation, such illustrative fortimicin factors as fortimicins A, B, C, D, E, KE, KF, KG, AH, AI, and derivatives thereof, such as the 4-N-substituted fortimicin derivatives, the 6'-N-substituted fortimicin derivatives, the 2'-N-substituted fortimicin derivatives, and the 1-, 2-, 5-, and/or 6'- modified fortimicin derivatives, e.g., 1-epi-fortimicin A, 2-epi-fortimicin A, 2-deoxyfortimicin A, 1-epi-2-deoxyfortamicin A, 2'-N-glycylfortimicin A, 6'-epi-fortimicin A, 4-N-(L-2-hydroxy-4-aminobutyl)-fortimicin B, 2-amino-2-deoxyfortimicin A, 2-amino-2-deoxy-2-epi-fortimicin A, 6'-N-methyl-fortimicin A, 2-deoxy-4-N-beta-aminoethylfortimicin B, 2,3-di-epi-fortimicin A and other O-methyl-containing fortimicin derivative compounds.

As used herein, the term "aminoglycoside" is intended to cover both the free base of such compounds and the nontoxic, pharmaceutically acceptable acid addition salts of such compounds. Suitable salts can be prepared in situ during the final isolation and purification of the aminoglycoside starting materials or O-demethylated products of the invention, or by reacting the free base with a suitable organic or inorganic acid. Illustrative salts include hydrochloride, hydrobromide, sulfate, bisulfate, acetate, haloacetate, oxalate, valerate, oleate, palmitate, stearate, laurate, borate, benzoate, lactate, phosphate, tosylate, citrate, maleate, fumarate, succinate, tartrate and napsylate salts, and the like. In a presently preferred embodiment of the invention, the trifluoroacetate acid addition salt of the aminoglycoside to be O-demethylated is used as a starting material, although other salts and/or the free base may be used for this purpose.

In accordance with the process of the invention, an O-methyl-containing aminoglycoside compound is reacted with a boron tris(haloacetate) to obtain the desired O-demethylated aminoglycoside derivative. The boron tris(haloacetate) compounds useful as reactants in the process of the invention include boron tris(trichloroacetate), boron tris(trifluoroacetate), boron tris(tribromoactate), boron tris(triiodoacetate), boron tris(dichloroacetate), boron tris(difluoroacetate), boron tris(dibromo-acetate), boron tris(diiodoacetate), and the like.

presently preferred reactants for this purpose include boron tris(trifluoroacetate) and boron tris(trichloroacetate), with a presently particularly preferred reactant being boron tris(trifluoroacetate).

The O-demethylation reaction may be conveniently carried out in a suitable solvent or suspending agent medium. A suitable solvent or suspending agent may be any solvent, mixture of solvents, suspending agent and/or mixture of suspending agents which does not react with the O-methyl-containing aminoglycoside or the boron tris(haloacetate) and which does not interfere with the O-demethylation reaction. Suitable solvents include, for example, acetic acid or a halogenated acetic acid, and when the O-demethylating agent is boron tris(trifluoroacetate), a presently preferred solvent medium is trifluoroacetic acid. The reaction may optionally be conducted under an inert atmosphere, such as under a nitrogen atmosphere. The use of an inert atmosphere is particularly desirable when a hygroscopic medium, such as a halogenated acetic acid, is employed as a solvent or suspending agent.

In order to optimize reaction yields, it is desirable to employ at least about 1.0 equivalents of the boron tris(haloacetate), more preferably at least about 1.2 equivalents of the boron tris(haloacetate), based upon the amount of aminoglycoside in the reaction mixture, although amounts of the boron tris(haloacetate) substantially in excess of 1.0 equivalents may be employed if desired.

The O-demethylation reaction may be carried out at temperatures of from about -15°C. to below the decomposition or degradation temperature of the aminoglycoside, more preferably from about 0°C. to about 50°C., and most preferably from about 20°C. to about 30°C. At relatively high temperatures, such as above about 72°C. in the case of fortimicin A, the aminoglycoside is subject to degradation, thereby limiting the ability to obtain the desired O-demethylated derivative. At temperatures below about -15°C., the O-demethylation reaction proceeds too slowly to be commercially practicable.

The reaction is allowed to proceed until a substantial amount of O-demethylation has occurred, preferably until at least about 60% conversion to the desired O-demethyl derivative has been obtained. The precise reaction time will depend upon many factors, including the nature of the aminoglycoside, the nature and amount of boron tris(haloacetate) employed, the nature of the reaction solvent, the reaction temperature, and other reaction conditions. In general, however, reaction times on the order of at least about 1 hour, more preferably at least about 5 hours, and most preferably at least about 24 hours are satisfactory.

In a presently preferred embodiment, the reaction mixture is formed by adding a solution of the aminoglycoside in a suitable solvent, e.g., trifluoroacetic acid, to a solution of the boron tris(haloacetate) in the solvent. The boron tris(haloacetate) solution may be formed by dissolving the boron tris(haloacetate) in the solvent. Alternatively, the boron tris(haloacetate) may be formed in situ by reacting a boron trihalide, such as $BBr_3$, $BCl_3$ and the like, with a haloacetic acid, such as trifluoroacetic acid, dichloroacetic acid, and the like. When the boron tris(haloacetate) is formed in this manner in situ, it is a presently preferred practice to maintain the temperature of the reaction mixture below about 15°C., and most preferably from about -10°C. to about 10°C., during formation of the boron tris(haloacetate). As a further alternative, the boron tris(haloacetate) may be formed in situ by adding a boron trihalide to a solution of the aminoglycoside in a haloacetic acid solvent, such as, e.g., trifluoroacetic acid, to complete the reaction mixture.

After the desired degree of O-demethylation has been obtained, the product may be recovered from the reaction mixture using conventional recovery techniques, such as by evaporation of excess boron tris(trihalo-acetate) and/or solvent, precipitation, crystalization, co-crystalization, extraction, chromatographic separation and the like. If desired, the product may optionally be subjected to additional purification techniques, such as chromatographic separation, and may be converted to the free base and/or to other pharmaceutically acceptable salts, in a conventional manner as will be apparent to those skilled in the art.

The foregoing may be more fully understood in connection with the following examples which are presented to illustrate, and not to limit, presently particularly preferred embodiments of the invention.

Example 1

Under an inert ($N_2$) atmosphere, 85 ml. of trifluoroacetic acid containing 24.35 g. (69.5 mmole) of boron tris(trifluoroacetate) was cooled to 0°C. To this mixture was added 10 g. (11.6 mmole) of fortimicin A tetra(trifluoroacetate), and the resulting mixture was stirred at 25°C. for 20 hours. The solvent was removed in vacuo at 40°C. in a water bath. The residue was dissolved in 30 ml. of methanol and the resulting solution evaporated to an oil in vacuo at 40°C. in a water bath. The residue was then again washed with two successive 30 ml. portions of methanol and evaporated to dryness to yield 11.0 g. of a white foam containing 73.1% 3-O-demethylformimicin A tetra(trifluoroacetate) (82% of theoretical yield).

### Example 2

Under an inert ($N_2$) atmosphere, 170 ml. of trifluoroacetic acid was cooled to 0°C., and 13.16 ml. (139.2 mmole) of boron tribromide was added over a two hour period. Fortimicin A tetra(trifluoroacetate) (20.0 g., 23.2 mmole) was dissolved in the reaction mixture and the mixture was stirred at 25°C. for 20 hours. The solvent was removed in vacuo at 40° in a water bath. The residue was dissolved in 50 ml. of methanol and the resulting solution evaporated to an oil in vacuo at 40°C. in a water bath. The residue was then again washed with two successive 50 ml. portions of methanol and evaporated to dryness to yield 18.1 g. of a white foam containing 81.2% 3-O-demethylfortimicin A tetra(trifluoroacetate) (92% of theoretical yield).

### Example 3

1.0 g. (1.7 mmole) of fortimicin A disulfate was dissolved in 40 ml. of trifluoroacetic acid and the solution was cooled to 0°C. under an inert ($N_2$) atmosphere. To this solution was slowly added 3.5 ml. (36.6 mmole) of boron tribromide. The mixture was stirred at 25°C. for 20 hours and then the solvent was removed in vacuo at 40°C. in a water bath. To the residue was added 30 ml. of methanol and the resulting solution evaporated in vacuo at 40°C. in a water bath to yield an oil. The residue was then again washed with two successive 30 ml. portions of methanol and evaporated to dryness to yield 1.15 g. of a white foam containing 40.2% 3-O-demethylfortimicin A disulfate (46% of theoretical yield).

### Example 4

17.1 g. (104.9 mmole) of trichloroacetic acid was dissolved in 50 ml. of methylene dichloride and the solution was cooled to -10°C. under an inert ($N_2$) atmosphere. To this solution was slowly added 3.3 ml.

(34.8 mmole) of boron tribromide, and the resulting mixture was stirred at 25°C. for one hour. The solvent was removed in vacuo at 40°C. in a water bath. The resulting residue was dissolved in 40 ml. acetic acid. Fortimicin A tetra(trifluoroacetate) (5.0 g., 5.8 mmole) was added to the reaction mixture and the mixture was stirred at 25°C. for 20 hours. The solvent was removed in vacuo at 40°C. in a water bath. To the residue was added 30 ml. of methanol and the solution was evaporated in vacuo at 40°C. in a water bath to yield an oil. The residue was then again washed with two successive 30 ml. portions of methanol and evaporated to dryness to yield 6.2 g. of a yellow foam containing 7.8% 3-O-demethyl-fortimicin A acetate (19% of theoretical yield).

Example 5

21 ml. (269 mmole) of trifluoroacetic acid was dissolved in methylene dichloride and the solution was cooled to -10°C. under an inert ($N_2$) atmosphere. A solution of 10.5 g. of boron trichloride in 50 ml. of methylene dichloride was slowly added to the trifluoroacetic acid solltion, and the resulting mixture was stirred at 25°C. for one hour. The solvent was removed in vacuo at 40°C. in a water bath, and the resulting residue was dissolved in 85 ml. trifluoroacetic acid. Fortimicin B free base (5.0 g., 15.0 mmole) was dissolved in the reaction mixture and the mixture was stirred for 20 hours at 25°C. The solvent was removed in vacuo at 40°C. in a water bath. To the residue was added 30 ml. of methanol and the solution evaporated in vacuo at 40°C. in a water bath to yield an oil. The residue was then again successively washed in 30 ml. portions of methanol and evaporated to dryness to yield 12.1 g. of white foam containing 83% 3-O-demethylfortimicin B tetra(trifluoroacetate) (85% of theoretical yield).

## Example 6

Under an inert ($N_2$) atmosphere, 200 ml. of trifluoroacetic acid was cooled to 0°C. To this solution was slowly added 3.5 ml. (37.3 mmole) of boron tribromide. 5.0 g. (6.2 mmole) of fortimicin E tetra-trifluoroacetate was dissolved in the reaction mixture and the mixture was stirred at 25°C. for 20 hours. The solvent was removed in vacuo at 40°C. in a water bath. The residue was dissolved in 50 ml. of methanol and the resulting solution was evaporated to an oil in vacuo at 40°C. in water bath. The residue was then again washed with two successive 50 ml. portions of methanol and evaporated to dryness to yield 5.39 g. of a white foam containing 35% 3-O-demethylfortimicin E tetratrifluoro-acetate (38% of theoretical yield).

## Example 7

Using the procedures of Examples 1, 2, 3 or 4, but replacing the salt of fortimicin A with a pharmaceutically acceptable salt of 1-epi fortimicin A, 2-epi-fortimicin A, 2-deoxyfortimicin A, 1-epi-2-deoxyfortimicin A, 2'-N-glycylfortimicin A, 6'-epi-fortimicin A, 4-N-(L-2-hydroxy-4-aminobutyl)-fortimicin B, 2-amino-2-deoxyfortimicin A, 2-amino-2-deoxy-2-epi-fortimicin A, 6'-N-methylfortimicin A, 2-deoxy-3-N-beta-aminoethylfortimicin B, or 2,3-di-epi-fortimicin A, the corresponding 3-O-demethlated fortimicin compound is obtained.

## Example 8

Using the procedure of Examples 1, 2, 3 or 4, but replacing the salt of fortimicin A with a pharmaceutically acceptable salt of istamycin B, sannamycin A, dactimicin, sporaricin A, or seldomycin factor 5, the corresponding O-demethylated compound is obtained.

While the inventive concepts have been described herein in connection with certain presently particularly preferred, illustrative embodiments, various modifications may be apparent to those in the art. Any such modifications are intended to be within the scope of the appended claims except insofar as precluded by the prior art.

CLAIMS:

1. A method of O-demethylating an aminoglycoside compound comprising reacting an O-methyl-containing aminoglycoside with a boron tris(haloacetate) for an amount of time sufficient to O-demethylate at least a portion of the aminoglycoside.

2. The method of Claim 1 wherein said tris(haloacetate) is selected from the group consisting of boron tris(trichloroacetate), boron tris(trifluoroacetate), boron tris(tribromoactate), boron tris(triiodoacetate), boron tris(dichloroacetate), boron tris(difluoroacetate), boron tris(diiodoacetate) and boron tris(dibromoacetate).

3. The method of Claim 1 wherein said boron tris(haloacetate) is selected from the group consisting of boron tris(trichloroacetate) and boron tris(trifluoroacetate).

4. The method of Claim 1 wherein the O-methyl-containing compound is an O-methyl-containing fortimicin.

5. The method of Claim 4 wherein the fortimicin is selected from the group consisting of fortimicin A, fortimicin B, 2-deoxyfortimicin A, 1-epi-fortimicin A, 2-epi-fortimicin A, 1-epi-2-deoxyfortimicin A, 2'-N-glycylfortimicin A, 6'-epi-fortimicin A, 4-N-(L-2-hydroxy-4-aminobutyl)-fortimicin B, 2-amino-2-deoxyfortimicin A, 2-amino-2-deoxy-2-epi-fortimicin A, 6'-N-methylfortimicin A, 2-deoxy-4-N-beta-aminoethyl-fortimicin B, and 2,3-di-epi-fortimicin A.

6. The method of Claim 4 wherein the O-methyl containing compound is fortimicin A.

7. The method of Claim 4 wherein the O-methyl containing fortimicin compound is forticimin A and the boron tris(haloacetate) is boron tris(trifluoroacetate).

8. The method of Claim 1 which further comprises recovering the O-demethylated aminoglycoside from the reaction mixture.

9. The method of Claim 8 which further comprises purifying the O-demethylated aminoglycoside by chromatographic separation.

10. The method of Claim 1 which further comprises forming the boron tris(haloacetate) in situ prior to reacting the O-methyl-containing aminoglycoside with the boron tris(haloacetate).

11. The method of Claim 10 wherein the boron tris(haloactate) is formed by reacting a haloacetic acid with a boron trihalide.

12. The method of Claim 11 wherein the haloacetic acid is trifluoroacetic acid and the boron trihalide is boron tribromide.

13. The method of Claim 12 wherein the trifluoroacetic acid is reacted with boron tribromide at a temperature of from about -10°C. to about 10°C.

14. The method of Claim 11 wherein the trifluoroacetic acid is stirred with boron tribromide under a nitrogen atmosphere for a period of at least about 2 hours.

15. The method of Claim 1 wherein the O-methyl-containing aminoglycoside is reacted with the boron tris(haloacetate) for a period of at least about five hours.

16. The method of Claim 1 wherein the O-methyl-containing aminoglycoside is reacted with the boron tris(haloacetate) for a period of at least about 24 hours.

17. The method of Claim 1 wherein the O-methyl-containing aminoglycoside is reacted with the boron tris(haloacetate) at a temperature of from about -15°C. to just below the decomposition temperature of the aminoglycoside.

18. The method of Claim 1 wherein the O-methyl-containing aminoglycoside is reacted with the boron tris(haloacetate) at a temperature of about 0° to about 50°C.

19. The method of Claim 1 wherein the aminoglycoside is reacted with the boron tris(haloacetate) for a sufficient amount of time to obtain at least about 60% O-demethylation of the aminoglycoside.

20. The method of Claim 1 wherein the aminoglycoside is reacted with the boron tris(haloacetate) for a sufficient amount of time to obtain at least about 90% O-demethylation of the aminoglycoside.

21. The method of Claim 8 which further comprises removing excess boron tris(haloacetate) from the reaction mixture prior to recovering the O-demethylated aminoglycoside.

22. The method of Claim 21 wherein the excess boron tris(haloacetate) is removed from the reaction mixture by evaporating the reaction mixture substantially to dryness.

23. A method of producing 3-O-demethyl-fortimicin A comprising reacting fortimicin A with a boron tris(haloacetate) for a sufficient amount of time to convert at least a portion of the fortimicin A to 3-O-demethylfortimicin A.

24. A method of producing 3-O-demethyl-fortimicin A comprising reacting the trifluoroacetate salt of fortimicin A with a boron tris(haloacetate) for a sufficient amount of time to convert at least a portion of the fortimicin A to 3-O-demethylfortimicin A.

25. The method of Claim 24 wherein the boron tris(haloacetate) is boron tris(trifluoro- acetate).

26. The method of Claim 24 which further comprises recovering 3-O-demethylfortimicin A from the reaction mixture.

27. The method of Claim 24 wherein the fortimicin A is reacted with the boron tris(haloacetate) at a temperature of about -15° to about 72°C.

28. The method of Claim 27 wherein the fortimicin A is reacted with the boron tris(haloacetate) for a sufficient period of time to convert at least about 60% of the fortimicin A to 3-O-demethyl-fortimicin A.

29. The method of Claim 27 wherein the fortimicin A is reacted with the boron tris(haloacetate) for a sufficient period of time to convert at least about 90% of the fortimicin A to 3-O-demethyl-fortimicin A.

30. The method of Claim 24 wherein the fortimicin A is reacted with the boron tris(haloacetate) at a temperature of about 0° to about 50°C.

31. The method of Claim 30 wherein the fortimicin A is reacted with the boron tris(haloacetate) for a sufficient amount of time to convert at least about 60% of the fortimicin A to 3-O-demethyl-fortimicin A.

32. The method of Claim 30 wherein the fortimicin A is reacted with the boron tris(haloacetate) for a sufficient amount of time to convert at least about 90% of the fortimicin A to 3-O-demethyl-fortimicin A.

33. The method of Claim 24 which further comprises forming the boron tris(haloacetate) in situ prior to reacting the fortimicin A with the boron tris(haloacetate).

34. The method of Claim 33 wherein the boron tris(haloacetate) is prepared by reacting a haloacetic acid with a boron trihalide.

35. The method of Claim 33 wherein the boron tris(haloacetate) is prepared by reacting a haloacetic acid with a boron trihalide at a temperature of about -10°C. to about 10°C.

36. The method of Claim 35 wherein the haloacetic acid is trifluoroacetic acid and the boron trihalide is boron tribromide.

37. A method of producing 3-O-demethyl-fortimicin A, comprising:

(a) adding boron tribromide to trifluoroacetic acid to form a reaction mixture,

(b) maintaining the reaction mixture at a temperature of about -10° to about 10°C. under a nitrogen atmosphere for a period of at least about 2 hours,

(c) adding fortimicin A tetra(trifluoro-acetate) to the reaction mixture,

(d) maintaining the reaction mixture at a temperature of about 5° to about 50°C. for a sufficient amount of time to convert at least about 60% of the fortimicin A tetra(trifluoroacetate) to 3-O-demethyl-fortimicin A tetra(trifluoroacetate), and then

(e) recovering 3-O-demethylfortimicin A tetra(trifluoroacetate) from the reaction mixture.

0106231

European Patent Office

EUROPEAN SEARCH REPORT

Application number

EP 83 10 9725

## DOCUMENTS CONSIDERED TO BE RELEVANT

| Category | Citation of document with indication, where appropriate, of relevant passages | Relevant to claim | CLASSIFICATION OF THE APPLICATION (Int. Cl. $^3$) |
|---|---|---|---|
| Y | EP-A-0 048 549 (ZAIDANHOJIN) * Pages 12,13 * | 1 | C 07 H 15/22 |
| Y | EP-A-0 035 337 (ABBOTT) * Page 6 * | 1 | |
| Y | GB-A-2 045 247 (ABBOTT) * Page 1 * | 1 | |
| Y | US-A-4 242 503 (P.A. LARTEY) * Claims 1,2 * | 1 | |
| Y | US-A-4 220 756 (J. KLOSS) * Claims 1,2 * | 1 | |
| Y | M. FIESER AND L.F. FIESER: "Reagents for organic synthesis, vol. 4, page 46, John Wiley & Sons, New York, US "Boron tris(trifluoroacetate) (BTFA)" * Page 46 * | 1 | TECHNICAL FIELDS SEARCHED (Int. Cl. $^3$) <br><br> C 07 H 15/00 |
| Y | CHEMICAL ABSTRACTS, vol. 84, 1976, page 507, no. 165190j, Columbus, Ohio, US & JP - A - 75 126 616 (SAGAMI CHEMICAL RESEARCH CENTER) 04-10-1975 * Abstract * | 1 | |

The present search report has been drawn up for all claims

| Place of search | Date of completion of the search | Examiner |
|---|---|---|
| THE HAGUE | 08-12-1983 | VERHULST W. |